# EUROPEAN PATENT SPECIFICATION

(11) **EP 3 485 820 B1**
(45) Date of publication and mention of the grant of the patent: **30.12.2020**
(21) Application number: 18213883.4
(22) Date of filing: 29.11.2017
(51) Int. Cl.: A61B 17/08, A61B 17/00

(54) **MAGNETIC WOUND CLOSURE DEVICE**
MAGNETISCHE WUNDVERSCHLUSSVORRICHTUNG
DISPOSITIF DE FERMETURE DE PLAQUE MAGNÉTIQUE

(30) Priority: 16.12.2016 US 201615381444; 16.06.2017 US 201715625703
(43) Date of publication of application: 22.05.2019
(62) Document number(s) of the earlier application(s) in accordance with Art. 76 EPC: 17204427.3 / 3 335 643
(73) Proprietor: Moustafa, Moustafa, Beverly Hills, CA 90212 (US)
(72) Inventor: Moustafa, Moustafa, Beverly Hills, CA 90212 (US)
(74) Representative: Hellmich, Wolfgang

(56) References cited:
- WO-A1-2016/091833
- WO-A2-2006/110420
- US-A1- 2007 060 951
- US-A1- 2014 214 078
- US-A1- 2015 088 195

## Description

The present invention relates generally to a device useful for wound healing, and more particularly to a magnetic device that draws wound edges in proximity to each other.

Wounds usually occur when there is trauma to the skin and underlying tissue. Types of trauma include lacerations, abrasions, incisions, punctures, and penetrations. After trauma, the wound begins to heal in a complex series of biochemical processes occurring in several wound-healing phases. The phases of healing are often categorized into a hemostasis phase, an inflammatory phase, a proliferative phase and a remodeling phase. In hemostasis, active bleeding is controlled by clotting. In the inflammatory phase, pathogens are removed by the body away from the wounded area, and biological factors are released (which later cause the division of cells involved in the proliferative phase). In the proliferative phase, new blood vessels are formed and wound contraction occurs. Also in the proliferative phase, epithelial cells cover the wound, providing an area of growth for new tissue. During contraction, the wound is made smaller by myofibroblasts attaching to the wound edges, and finally, during the remodeling phase, collagen fibers are realigned along tension lines formed during the earlier phases of healing.

Not only is the process of wound healing complex, it is also fragile because many factors can lead to a disruption of proper wound healing, including re-injury of the tissue, bacterial infection, and physical stress on the damaged tissue. A variety of devices and methods have been used to aid in the wound healing process. These devices and methods are generally divided into one of three types: primary intention, secondary intention, and tertiary intention. The primary intention devices and methods bring the edges of the wounds together, so that the edges are reapproximated. Reapproximation helps to minimize scarring, and increases the speed at which wound contraction and healing occur. Examples of primary intention devices and methods include the use sutures, staples, tape, glue, and hooks. Primary intention techniques to heal wounds are the most common techniques used by practitioners.

While not as commonly used, secondary intention devices and methods include first allowing the wound to first granulate without closing the wound. The wound is packed and drained, often daily, to remove debris and encourage granulation tissue formation. Still less common are tertiary intension devices and methods, which delay the closure of the wound even longer, so that the practitioner can close the wound at a later time. Tissue grafting is an example of a tertiary method for wound healing.

Wound edge reapproximation is key to wound healing. If the edges of the wound are not immediately reapproximated soon after injury, healing may be delayed. This delay in healing may leads to scarring and infection. While in some circumstances a delay is advantageous, practitioners generally want to close an open wound as soon as possible.

The traditional method to reapproximate wound edges is by sutures, where a practitioner stitches a threading material to connect opposing sides of a wound. Sutures and suturing techniques are well known in the prior art, such as described by Fällman in U.S. Patent No. 8,267,959. Other devices and methods to reapproximate wound edges include hooking devices, such as the hook closure device disclosed by Bargon in U.S. Patent Application No. 13/266,825, where a band placed over a wound has a multiplicity of hook elements that engage a mesh on the opposing side of a wound.

The use of adhesive strips is another method to aid in wound closure. In U.S. Patent No. 4,825,866 to Pierce, adhesive strips are placed on opposite sides of a wound and drawn together to reapproximate the wound edges. Stapling and clipping the edges of wound are other techniques to reapproximate wound edges, as described by Zadno in U.S. Patent No. 7,556,632.

The use of magnets to reapproximate wound edges also has been previously described. U.S. Patent Application No. 10/512,964 to Wheatley and U.S. Patent No. 9,402,605 to Viola are two applications that have described tissue-joining devices comprising interconnecting components where the magnetic components are attracted to each other and draw tissue together using magnets attached to a patient's skin. Flexible magnetic devices have improved the ability to place magnets on wounds where either the wound itself is non-linear or where the wound is located on a curved surface. United States Patent No. 9,486,217 to Moustafa discloses the use of flexible magnets to reapproximate wound edges that are non-linear or on a curved surface.

WO 2016/091833 A1 discloses a medical dressing for achieving an invisible or less visible scar and method of use. The medical dressing for wound skin, consisting of a pair of adhesive tapes, juxtaposed to apply on both sides of the wound to be healed, momentarily separated until the end of exposure, by a key having the form of a flat element or removable strip. Further, the medical dressing comprises ribbons which are juxtaposed and intended to attract magnetically. They form an assembly secured temporarily by a key system in the form of a pressed element and held on both sides by magnets.

US 2014/214078 A1 discloses a magnetic wound closure assembly and method of use. The assembly has a flexible sheet with two magnets, one magnet on each of two ends of the flexible sheet. The magnets surround the ends of the flexible sheet, such that the magnets cannot be pulled off from the ends in a perpendicular direction relative to the plane of contact surface of each magnet. The magnets cannot be removed merely by pulling the flexible sheet away from the magnets because of at least one protrusion on each magnet directed toward the central axis of each magnet.

Other compositions and methods to reapproximate wound edges include the use of medical adhesives, such as cyanoacrylate glues that provide for very tight, high-strength closure of wounds without the need for the physical closure accomplished with sutures. However, cyanoacrylate based glues have been associated with the formation of toxic byproducts, and even non-toxic versions are generally only useful for smaller, shallow lacerations in low-tension areas. These adhesives can be very unforgiving if the practitioner needs to remove the glue. Another disadvantage of using glues for wound clos3ure is that leakage of glues can cause serious ramifications, especially if the adhesives are toxic and the wounds are near sensitive anatomical structures, such as the eye. Still another disadvantage is that adhesives can trap pathogens and other particles within the wound.

Each type of wound closure device and technique has advantages and disadvantages. Sutures pose the risk of injury to the patient caused by the needle as well as to health care professions. The process of suturing also can take a substantial amount of time depending on the size of the wound. Using staples for wound closure is more rapid than suturing, however, unlike sutures, which may be absorbed by the body, staples usually have to be removed by a special tool. Sutures and staples also require applying local anesthesia, which could be dangerous to the patient, especially in the event of an allergic reaction. Furthermore, if the practitioner needs to enter the wound area, the sutures or staples need to be cut or removed, and both sutures and staples can lead to scarring.

Complex wound closure devices that reduce some of these disadvantages have many individual parts, are difficult to apply, or are expensive. Accordingly, it would be advantageous to make available a novel wound closure device that reduces these stated disadvantages and there remains a need for quick and easy to use devices and methods to reapproximate wound edges.

The present invention relates to a wound closure device, which lead to improved wound healing. It is an object of the present invention to provide a magnetic wound closure device, such that when two magnets are attached to skin on opposites sides of a wound, the magnets attract each other, thereby pulling together the edges of the wound. Before the magnets reapproximate the wound edges, the magnets are separated by a partition on a peelable removable sheet so that the practitioner can place the device on the patient in a precise location before the magnets are drawn together. Once the removable sheet is removed from the magnet, the magnets draw in toward each other and pull in the skin edges around the wound. There are several advantages to the described invention, including: 1) placement of the device on a patient is faster than using staples or sutures, 2) no need to wait for an anesthetic, 3) less trauma, 4) no painful injections are needed, 5) inspection of the wound is simple because the device is easily removable, and 6) the device can be easily manufactured in a variety of sizes and shapes that accommodate various wound sizes.

The magnetic wound closure device has a first magnet and a second magnet. The first magnet and second magnet are characterized as elongated magnets each having a top surface, a bottom surface, an inner edge, and an outer edge. The inner edge of the first magnet and the inner edge of the second magnet are opposite polarities of each other. The device also includes a removable sheet having an elongated partition member. The removable sheet is designed to indirectly connect the first magnet to the second magnet by adhering to the top surface of the first magnet and top surface of the second magnet. The removable sheet has a top surface and a bottom surface. The bottom surface of the removable sheet is removably affixed to the top surfaces of the two magnets. The elongated partition member extends from the bottom surface of the removable sheet and is designed to be positioned between the inner edges of the two magnets to separate them. The elongated partition member thereby prevents the inner edge of the magnets from directly contacting each other when the wound closure device is being placed along the edges of a wound. When the removable sheet and elongated partition member are removed from the magnets, the magnets are magnetically drawn to each other, causing the inner edges of each magnet to move together, thereby reapproximating wound edges.

Another embodiment of the invention is where the bottom surfaces of the magnets have an adhesive layer to affix the magnets to the skin of a patient. The adhesive layer may be covered be an adhesive covering before being removed and placed on a patient. The removable sheet may be peelable and also have an adhesive layer on its bottom surface for affixing the removable sheet to the first and second magnet.

Another embodiment of the invention is where the magnets include a protective covering, such as a polymer enclosure.

In a further embodiment of the invention, the bottom surface of the removable sheet is sized and shaped substantially the same as the top surfaces of the magnets when the magnets are aligned together along their respective inner surfaces. The elongated partition member is positioned substantially along the middle of the longitudinal axis of the removable sheet.

In another embodiment of the invention, to prevent the inner edges of the magnets from shifting along the longitudinal axis, each inner edge has a plurality of notches and protrusions. The notches on each inner edge are complementary shaped to protrusions on the opposing magnet, so that when the inner edges of each magnet are aligned with each other, the complementary notches and protrusions reduce the possibility of movement of the magnets along their respective inner edges. When the magnets are in proximity to each other, the inner edges move toward each other, thereby drawing the skin under the magnets to each other and reapproximating wound edges.

In another embodiment, the plurality of notches and protrusions are serrations, and the serrations can be V-shaped serrations.

In another embodiment of the invention, the first magnet has a first flexible enclosure and the second magnet has a second flexible enclosure. Each enclosure is filled with a powdered magnetic material. The magnetic powder within the enclosures allow the magnets to maintain either a linear or non-linear shape even after they are placed on the patient. The place of the first and the second magnet on opposing edges of a non-linear wound allow the magnets to attract each other along a non-linear inner edge of the first magnet and a non-linear edge of the second magnet, thereby reapproximating wound edges of a non-linear wound.

These and other features and advantages of the present invention will become appreciated, as the same becomes better understood with reference to the specification, claims and drawings herein:
**FIG. 1** is a bottom perspective view of an embodiment of a wound closure device.
**FIG. 2** is a top perspective exemplary view of the embodiment of Fig. 1 placed on an arm.
**FIG. 3** a top perspective exemplary view of the embodiment of Fig. 1 as the removable layer is being peeled off and magnets still separated.
**FIG. 4** is a top perspective exemplary view of the embodiment of Fig. 1 after the removable layer has been completely removed and the inner edges of the magnets have reapproximated the wound edges.
**FIG. 5** is a perspective view of a wound closure device having notched and protruding inner edges along two separated magnets.
**FIG. 6** is a perspective view of the wound closure device of Fig. 5 where the inner edges along the two magnets have been magnetically attracted to each other and connected.
**FIG. 7** is a top exemplary view of an embodiment where the wound closure device is made of two flexible tubes of powdered magnet material to easily curve around non-linear wound edges before the magnets attract together.
**FIG. 8** is a top exemplary view of FIG. 7 after the magnets have attracted together to close the wound.

The invention now will be described more fully hereinafter with reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art.

It will be understood that when an element is referred to as being "on" another element, it can be directly on the other element or intervening elements may be present there between. As used herein, the term "and/or" includes any and all combinations of one or more of the associated listed items.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another element, component, region, layer or section.

As used herein, the singular forms "a," "an," and "the," are intended to include the plural forms as well, unless the context clearly indicates otherwise. It will be further understood that the terms "comprises" and/or "comprising," "includes" and/or "including," and "have" and/or "having," when used in this specification, specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom," and "upper" or "top," and "inner" or "outer," may be used herein to describe one element's relationship to another element as illustrated in the Figures. It will be understood that relative terms are intended to encompass different orientations of the device in addition to the orientation depicted in the Figures.

Unless otherwise defined, all terms used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments of the present invention are described herein with reference to idealized embodiments of the present invention. As such, variations from the shapes of the illustrations as a result, for example, of manufacturing techniques and/or tolerances, are to be expected. Thus, embodiments of the present invention should not be construed as limited to the particular shapes of regions illustrated herein but are to include deviations in shapes that result, for example, from manufacturing.

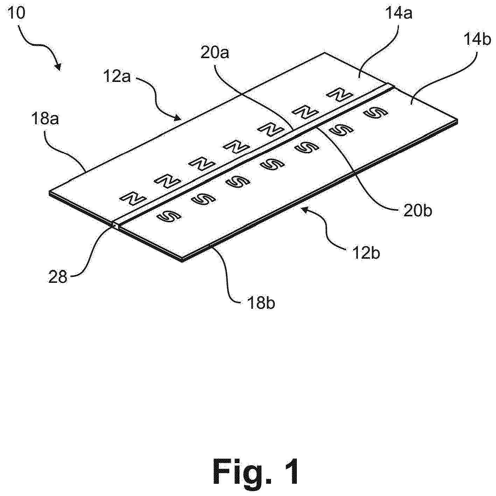

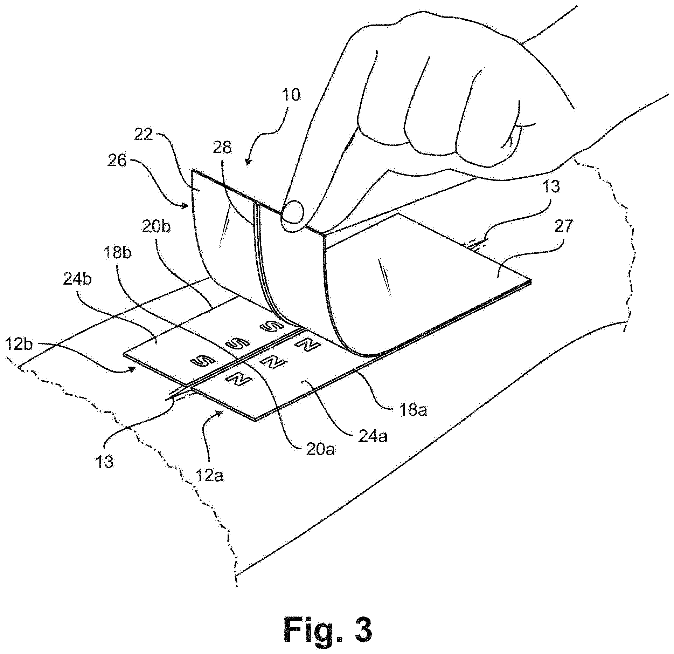

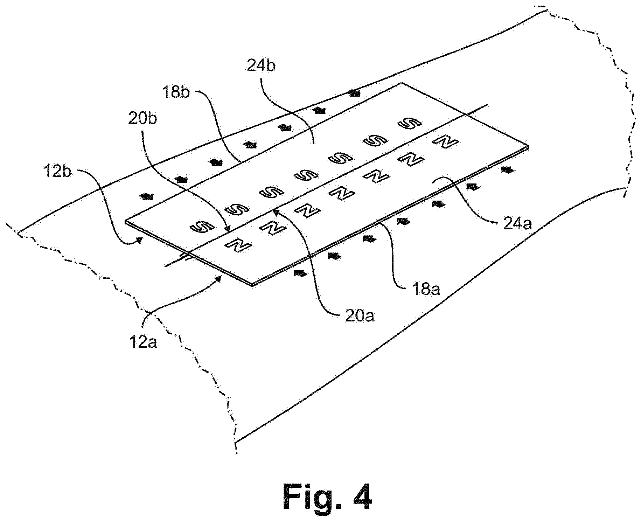

A wound closure device is provided for closing wounds without sutures. **FIGs. 1-4** depict one embodiment of a wound closure device **10** and how the device **10** is used on a patient. The wound closure device **10** includes a first magnet **12a** and a second magnet **12b.** The magnets **12a, 12b** are flexible elongated strips capable of flexing along the longitudinal axis, such as shown in **Fig. 3****.** Each magnet has a top surface **24a, 24b,** a bottom surface **14a, 14b,** an outer edge **18a, 18b,** and an inner edge **20a, 20b.** The inner edges **20a, 20b** have opposite magnetic polarity. For example, if the inner edge **20a** of the first magnet **12a** is north, then the inner edge of **20b** of the second magnet **12b** is south, and vice versa.

Attached to the magnets **12a, 12b** is a removable sheet **26** having a top surface **27** and bottom surface **22.** The removable sheet **26** can be made of any flexible material, including polymers, polyvinyls, elastomers, thermoplastics, polyolefins, polyesters, or the like. The removable sheet indirectly connects the first magnet 12a to the second magnet **12b** by adhering the top surfaces **24a,** 24b of the first and second magnets **12a, 12b** to the bottom surface **22** of the removable sheet **26.** The bottom surface **22** of the removable sheet **26** is removably affixed to the top surfaces **24a, 24b** of the magnets **12a, 12b.**

Extending from the bottom surface **22** of the removable sheet **26** is an elongated partition member **28.** The partition member **28** may be made of the same material as the removable sheet **26** and may be integral with the sheet **26** or attached to the sheet **26.** The elongated partition member **28** runs substantially down the longitudinal axis of the removable sheet **26** and is designed to be positioned between and separate the two magnets **12a, 12b** along their inner edges **20a, 20b.** By having the partition member **28** between the inner edges **20a, 20b,** the partition member **28** prevents the inner edge **20a** of the first magnet **12a** from directly contacting the inner edge **20b** of the second magnet **12b.** This configuration allows the practitioner to maneuver the device around the wound **13** when the magnets are not yet connected directly to each other so that the first magnet **12a** can be positioned on one side of the wound **13** and the second magnet **12b** can be positioned on the opposing side of wound **13,** with the wound **13** positioned between the two magnets **12a, 12b.**

The elongated partition member **28** may have a variety of structures and in on embodiment is an elongated tab having a width of less than 2 cm, a depth of less than 2 cm. The wider the partition member **28** is, then the larger the wound **13** that can be fit between the magnets **12a, 12b.** However, if the partition member **28** is too wide, the magnets will be two far apart to attract each other once the partition member **28** is removed. If the partition member **28** is too thin, then the magnets **12a, 12b** would be placed directly on top of the wound and not on adjacent skin. In one embodiment, the depth of the partition member **28** is substantially the same as the depth of each magnet **12a, 12b,** so that the bottom of the partition member **28** is flush with the bottom surfaces of each magnet **12a, 12b** when the removable sheet **26** and magnets **12a, 12b** are connected together. In other embodiments, the width and depth of the elongated partition member **28** is less than 1 cm.

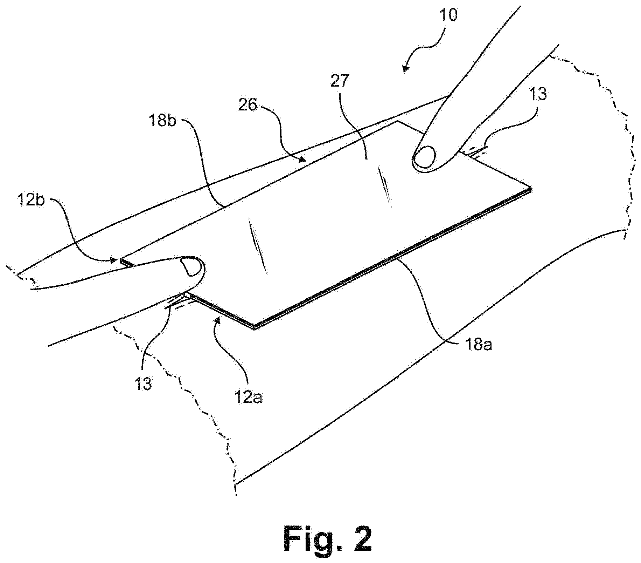

Once the device **10** is placed over the wound **13,** as shown in **Fig. 2****,** the practitioner removes the removable sheet **26,** as shown in **Fig. 3****,** by peeling the removable sheet **26** off of the magnets **12a, 12b.** In **Fig. 3****,** the magnets are still spaced apart from each other because the partition member **28** is still positioned between the magnets **12a, 12b.** When the removable sheet **26** and partition member **28** are peeled away from the magnets **12a, 12b,** the inner edges **20a, 20b** of the magnets **12a, 12b** are drawn together, thereby reapproximating wound edges by pulling the skin underneath the magnets **12a, 12b** together.

To aid in adhering the magnets to the skin, the bottom surfaces of the magnets **14a, 14b** may have an adhesive layer. The adhesive layer can be covered with a peelable cover (not shown) designed to be removed before affixing the magnets **12a, 12b** to the skin. The removable sheet **26** may also have an adhesive layer, or the top surfaces of the magnets **24a, 24b** may have an adhesive layer to affix the removable sheet **26** to the magnets **20a, 20b.** The adhesive layer is a weak adhesive layer, which keeps the sheet **26** affixed to the magnets, but allows the sheet **26** to be easily peeled away from the magnets once the device **10** is placed on the patient. In an alternative manner to apply the device to the patient's skin, instead of an adhesive layer on the magnet, a practitioner could apply an adhesive directly on the patient's skin around a wound, and thereafter place the device on the skin adhesive.

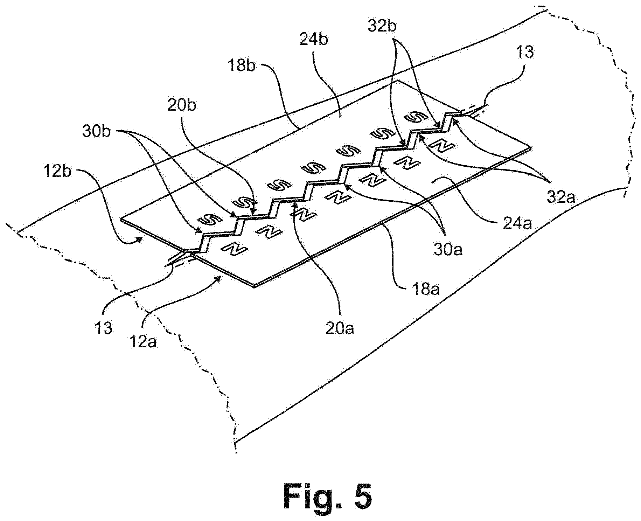

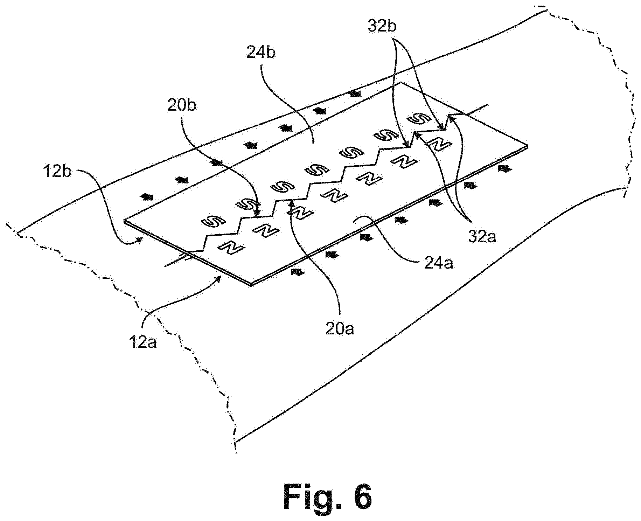

Turning to **Fig. 5** and **Fig. 6****,** in another embodiment of the device **10,** the inner edge of the first magnet **12a** is characterized as having a first plurality of notches **30a** and the inner edge of the second magnet **12b** is characterized as having a second plurality of notches **30b.** The notches **30a, 30b** can be any size or shape, but are complementary shaped so that the protrusions **32a** formed by the notches **30a** on the first magnet **12a** fit within the notches **30b** of the second magnet **12b,** and vice versa. When the device **10** is placed on opposite sides of the wound **13,** the magnets **12a, 12b** are spaced apart, as shown in **Fig. 5****.** After placement on opposite sides of a wound, the magnets **12a, 12b** attract each other so that the notches **30a, 30b** interlock with the protrusions **32a, 32b** on the opposing magnet, as shown in **Fig. 6****.** As shown in **Fig. 5** and **Fig. 6****,** the notches are V-shaped, but other shapes , such as square shaped, rectangular shaped, circular shaped, irregularly shaped, or any other shape where a complementary protrusion can fit within the notch of an opposing magnet.

The notches **30a, 30b** are advantageous because they prevent slippage and movement along the longitudinal axis of the inner edges due to the flanking effect of the arms of the notches and protrusions. Wound edge alignment is a desirable feature of wound closure because if the wound's edges are not perfectly aligned, the resulting healed wound may have a "dog ear" deformity, which would require surgical correction. The anti-slippage feature created by the notches and protrusions along the inner edges of the magnet prevents the skin from sliding underneath the magnets. Protrusions and notches can vary in size. In one embodiment, a protrusion tip is approximately 4 mm in height. In one embodiment, the length from protrusion tip to the adjacent protrusion tip is approximately 8 mm. In one embodiment, the length of the magnet is approximately 8 cm. In one embodiment, the thickness of the magnet is approximately 1 mm and the width of each magnet, from the tip of a protrusion to the outer edge **18a, 18b,** is approximately 2 cm. Differences in dimensions of notches and protrusions can be well tolerated such as notches and protrusions that are at least half or at least double the notch and protrusion disclosed above.

The dimensions of each magnet **12a, 12b** of device **10** can be of any length, but preferably between 3 cm and 10 cm in length, and 2 cm and 6 cm in width. Preferably, the height of each magnet **12a, 12b** is between 0.5 mm and 5 mm, and approximately 1 mm in height.

In another embodiment, each magnet **12a, 12b** is between 5 cm and 20 cm in length, between 1 cm and 6 cm in width, and between 0.5 mm and 10 mm in height. In another embodiment, the dimension of each magnet **12a, 12b** is approximately 4 cm in length, 2 cm in width, and 1 mm in height.

Preferably, the magnets have a protective covering or are enclosed within a housing, such as a polymer, enclosure. Enclosures for magnets in wound healing devices have been disclosed in other wound healing devices, as well as additional layers within the enclosure to aid in healing, such as: insulation layers, absorbent layers, polymer layers, holes that allow for drainage of fluids, adhesive layers, and removable covers, which are described and illustrated in **Fig. 1** and **Fig. 2** of U.S. Patent No. 9,486,217 to Moustafa.

The enclosure may be made of any one of number of polymers, but in a preferred embodiment is a silicone enclosure. Other materials for an enclosure may be made from natural or synthetic polymers including rubber, neoprene, polyvinyl chloride, polyvinyl butyral, polystyrene, polyethylene, polypropylene, nylon, polyacrylonitrile.

The magnets **12a, 12b** may be made from a number of materials, such as ferromagnetic materials, or rare-earth elements, such as magnets made from alloys of neodymium, iron and boron. The advantage of rare-earth magnets, such as neodymium magnets is that their crystalline structures have very high magnetic anisotropy and can retain high magnetic moments in the solid state.

In a preferred embodiment, the magnet is a flexible magnet that is rolled or extruded in a magnetic film, and then cut to size into a magnetic sheet or strip. In one magnet useful for wound closure, the magnet comprises NdFeB magnetic powder, chlorinated polyethylene (CPE), and an annexing agent such as soybean oil.

In another embodiment, the magnet in the device is comprised of approximately 90.5% NdFeB powder, 8.5% CPE, and 1% annexing agent. In yet another embodiment, NdFeB powder may be comprised of approximately 31.0-31.8% PrNd, approximately 64-66.5% Fe, approximately 1.00-1.03% B, approximately 1.5-1.8% Dy, approximately 0.5-0.8% Co, approximately 0-0.25% Nb, and approximately 0.0-0.2% Al. Deviations from the percentages above that also allow for a strong but flexible magnet are allowed and known by persons having ordinary skill in the art.

The adhesive used in the embodiments may be made from any one of a number of adhesive compositions, including: reusable adhesives, pressure sensitive adhesives, contact adhesives, resins, epoxies, polyurethane, cyanoacrylate (CA), polymers, acrylic-based adhesives that cure under ultraviolet (UV) light, silicone based adhesives, and polyolefinic polymers. A removable cover that protects the adhesive from the environment before use may be a peel-off tape. The practitioner removes the adhesive cover before placing the device 10 on the patient's skin.

The inclusion of plasticizers within the polymer composition of the enclosure lowers the glass transition temperature (T_{g}) of the polymer, therefore allowing device **10** to flex when the magnets **12a, 12b** within the polymer enclosure flexes. Plasticizers are commonly known and used in the art, and may include phthalate ester plasticizers commonly used in medical devices such as: dicarboxylic/triboxylic ester-based plasticizers, including Bis (2-ethylhexyl) phthalate, Di-n-butyl phthalate, or Diisooctyl phthalate.

Since no sutures or staples are used when applying the device **10,** the invention is advantageous for emergency situations when medical personnel are overloaded, or even in non-emergency situations for use with young children who would be more apprehensive regarding traditional wound closure devices and methods.

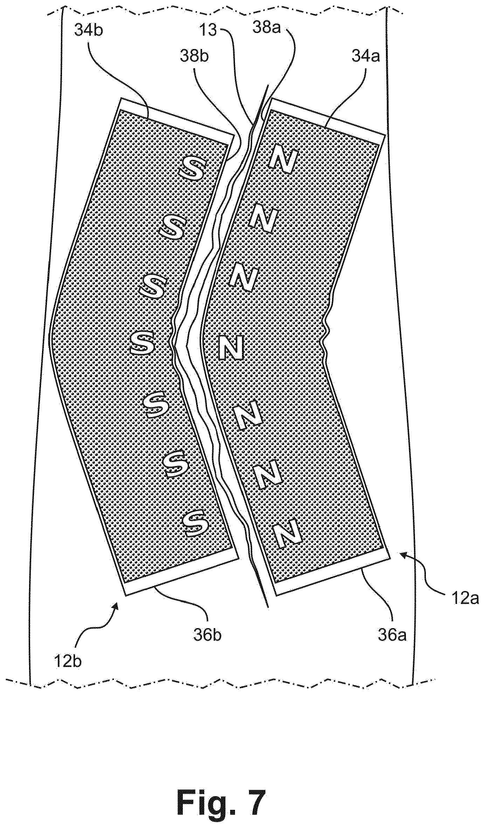

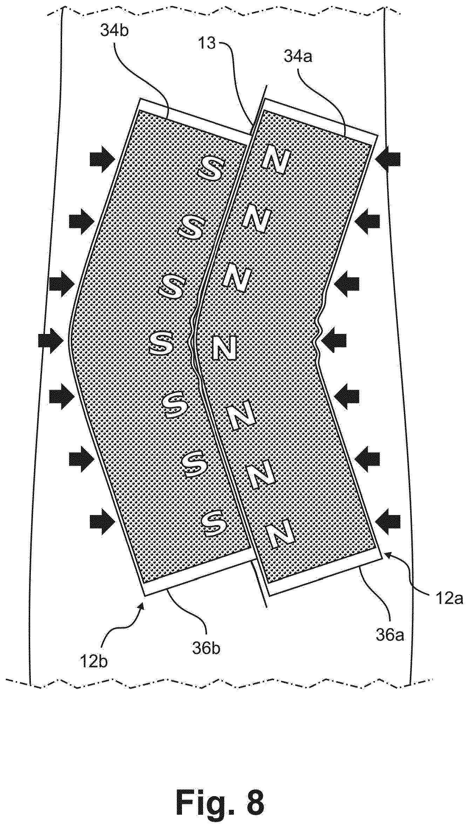

**Fig. 7** and **Fig. 8** illustrate another embodiment of the invention, where the magnets **12a, 12b** placed on opposite edges of a wound **13** are not magnetic strips but instead or are comprised of magnetic powdered material **34a, 34b** within a flexible enclosure **36a, 36b.** The flexible enclosures **36a, 36b** can be made of any thin, flexible material, such as plastic, polyethylene, latex, nitrile, or the like, and allow the magnets **12a, 12b** to be easily positioned on a patient to match the shape of the wound. The powder **34a, 34b** allows the magnets **12a, 12b** to maintain either a linear shape or non-linear shape after placement on a patient. In **Fig. 7** and **Fig. 8****,** the magnets **12a, 12b** are in a non-linear shape. Other types of flexible enclosures for magnetic powder may be envisioned. In one embodiment, the flexible enclosures **36a, 36b** are hollow cylindrical tubular structures, but the shapes and sizes of the flexible enclosures **36a, 36b** may vary.

Examples of magnetic powder **34a, 34a** that can fill the enclosures **36a, 36b** can be, but are not limited to: iron oxide, barium ferrite, strontium ferrite, magnetite, Iron (II,III) oxide, neodymium powder, rare earth magnet powder, or any other permanent magnet that can be powderized and placed within the flexible enclosures **36a, 36b.** **Fig. 7** depicts the magnets **12a, 12b** around the edges a non-linear wound before the magnets have attracted each other to reapproximate wound edges. Since the magnets **12a, 12b** are comprised of magnetic powder **34a, 34b,** the magnets **12a, 12b** can be easily bent or curved and maintain their shapes. **Fig. 8** depicts the magnets **12a, 12b** of **Fig. 7** after the inner edges **38a, 38b** of the magnets **12a, 12b** have attracted to each other, thereby reapproximating wound edges. The inner edges **38a, 38b** of the magnets **12a, 12b** in **Fig. 7** and **8** are shown as non-linear inner edges **38a, 38,** but can also be shaped to be straight linear inner edges to approximate linear wounds as well.

The magnets illustrated in **Figs. 7** and **8** may have the same additional layers of adhesives, covers, and flexible sheets as described in the previous embodiments.

## Claims

1. A magnetic wound closure device (10) comprising:
i) a first magnet (12a) and a second magnet (12b), the first magnet (12a) and the second magnet (12b) being elongated magnets each having a top surface (24a, 24b), a bottom surface (14a, 14b), an inner edge (20a, 20b; 38a, 38b), and an outer edge (18a, 18b), wherein the inner edge (20a; 38a) of the first magnet (12a) has an opposite polarity to the inner edge (20b; 38b) of the second magnet (12b);
ii) a removable sheet (26) having an elongated partition member (28), the removable sheet (26) designed to position the first magnet (12a) relative to the second magnet (12b) by adhering to the top surface (24a) of the first magnet (12a) and the top surface (24b) of the second magnet (12b),
wherein the removable sheet (26) has a top surface (27) and a bottom surface (22), wherein the bottom surface (22) of the removable sheet (26) is removably affixed to the top surface (24a) of the first magnet (12a) and the top surface (24b) of the second magnet (12b);
wherein the elongated partition member (28) extends from the bottom surface (22) of the removable sheet (26) and is designed to be positioned between, and to separate, the inner edge (20a; 38a) of the first magnet (12a) and the inner edge (20b; 38b) of the second magnet (12b), thereby preventing the inner edge (20a; 38a) of the first magnet (12a) from directly contacting the inner edge (20b; 38b) of the second magnet (12b) when the magnetic wound closure device (10) is being placed along edges of a wound (13);
wherein the elongated partition member (28) is an elongated tab having a width of less than 2 cm, a depth of less than 2 cm, and wherein the first magnet (12a) and the second magnet (12b) have a depth of less than 2 cm;
whereby the distance between the inner edge (20a; 38a) of the first magnet (12a) and the inner edge (20b; 38b) of the second magnet (12b) is less than 2 cm when the elongated tab is positioned between the inner edge (20a; 38a) of the first magnet (12a) and the inner edge (20b; 38b) of the second magnet (12b); and
whereby when the removable sheet (26) and the elongated partition member (28) are removed from the first and the second magnets (12a, 12b), the first and second magnets (12a, 12b) are magnetically drawn to each other, causing the inner edge (20a; 38a) of the first magnet (12a) to move toward the inner edge (20b; 38b) of the second magnet (12b), thereby reapproximating wound (13) edges.

2. The magnetic wound closure device (10) of claim 1, wherein the bottom surfaces (14a, 14b) of the first magnet (12a) and the second magnet (12b) each have an adhesive layer for affixing the first magnet (12a) and the second magnet (12b) to skin of a patient; and,
wherein the adhesive layer is protected by a removable adhesive layer cover designed to be removed before affixing the first magnet (12a) and the second magnet (12b) to skin of a patient.

3. The magnetic wound closure device (10) of claim 1 or 2, wherein the removable sheet (26) is a peelable sheet having an adhesive layer on the bottom surface (22) of the removable sheet (26) for affixing the removable sheet (26) to the first magnet (12a) and the second magnet (12b).

4. The magnetic wound closure device (10) of one of the preceding claims, wherein the first magnet (12a) and the second magnet (12b) are flexible magnets capable of flexing in a longitudinal direction; and,
wherein the removable sheet (26) is capable of flexing in a longitudinal direction.

5. The magnetic wound closure device (10) of one of the preceding claims, wherein the first magnet (12a) and the second magnet (12b) are each enclosed within a protective covering.

6. The magnetic wound closure device (10) of claim 5, wherein the protective covering is a polymer enclosure.

7. The magnetic wound closure device (10) of one of the preceding claims, wherein the elongated tab having a width of less than 1 cm, a depth of less than 1 cm, and wherein the first magnet (12a) and the second magnet (12b) each have a depth of less than 1 cm.

8. The magnetic wound closure device (10) of one of the preceding claims, wherein the bottom surface (22) of the removable sheet (26) is sized and shaped substantially the same as the top surfaces (24a, 24b) of the first and second magnets (12a, 12b) when the first and second magnets (12a, 12b) are oriented together along their respective inner edges (20a, 20b; 38a, 38b); and,
wherein the elongated partition member (28) is positioned substantially along the middle of the longitudinal axis of the removable sheet (26).

9. The magnetic wound closure device (10) of one of the preceding claims, wherein the first and second magnets (12a, 12b) are neodymium magnets.

10. The magnetic wound closure device (10) of one of the preceding claims, wherein each of the first and second magnets (12a, 12b) have dimensions in the range of between 5 cm and 20 cm in length and 1 cm and 6 cm in width.

11. The magnetic wound closure device (10) of one of the preceding claims, wherein:
the inner edge (20a; 38a) of the first magnet (12a) has a first plurality of notches (30a) and a first plurality of protrusions (32a), and the inner edge (20b; 38b) of the second magnet (12b) has a second plurality of notches (30b) and a second plurality of protrusions (32b) and is complimentarily shaped to the inner edge (20a; 38a) of the first magnet (12a);
whereby when the first plurality of notches (30a) of the first magnet (12a) are aligned with the second plurality of protrusions (32b) on the second magnet (12b), the first and second magnets (12a, 12b) interlock with each other only after the removable sheet (26) has been removed from the top surfaces (24a, 24b) of the first and second magnets (12a, 12b), wherein the first and second magnets (12a, 12b) prevent longitudinal slippage along the inner edges (20a, 20b; 38a, 38b) of the first magnet (12a) and the second magnet (12b) and
whereby wound edges are reapproximated when the magnetic wound closure device (10) is placed along wound edges of a patient by moving the inner edge (20a; 38a) of the first magnet (12a) toward the inner edge (20b; 38b) of the second magnet (12b).

12. The magnetic wound closure device (10) of claim 11, wherein the first plurality of notches (30a) and the first plurality of protrusions (32a) are serrations.

13. The magnetic wound closure device (10) of claim 11 or 12, wherein the first and second plurality of notches (30a, 30b) and the first and second plurality of protrusions (32a, 32b) are V-shaped.

14. The magnetic wound closure device (10) of one of claims 11 to 13, wherein the removable sheet (26) is designed to indirectly connect the first magnet (12a) to the second magnet (12b) by adhering to the top surface (24a) of the first magnet (12a) and the top surface (24b) of the second magnet (12b).

## Patentansprüche

1. Magnetische Wundverschlussvorrichtung (10), mit:
i. einem ersten Magneten (12a) und einem zweiten Magneten (12b), wobei der erste Magnet (12a) und der zweite Magnet (12b) längliche Magnete sind, die jeweils eine obere Oberfläche (24a, 24b), eine untere Oberfläche (14a, 14b), eine Innenkante (20a, 20b; 38a, 38b) und eine Außenkante (18a, 18b) aufweisen, wobei die Innenkante (20a; 38a) des ersten Magneten (12a) eine entgegengesetzte Polarität gegenüber der Innenkante (20b; 38b) des zweiten Magneten (12b) aufweist;
ii. einer ablösbaren Folie (26), die ein längliches Trennelement (28) aufweist, wobei die ablösbare Folie (26) so konstruiert ist, dass der erste Magnet (12a) gegenüber dem zweiten Magneten (12b) durch Anhaften an der oberen Oberfläche (24a) des ersten Magneten (12a) und an der oberen Oberfläche (24b) des zweiten Magneten (12b) positioniert wird,
wobei die ablösbare Folie (26) eine Oberseite (27) und eine Unterseite (22) aufweist, wobei die Unterseite (22) der ablösbaren Folie (26) ablösbar an der Oberseite (24a) des ersten Magneten (12a) und an der Oberseite (24b) des zweiten Magneten (12b) befestigt ist;
wobei sich das längliche Trennelement (28) von der Unterseite (22) der ablösbaren Folie (26) erstreckt und so konstruiert ist, dass es zwischen der Innenkante (20a; 38a) des ersten Magneten (12a) und der Innenkante (20b; 38b) des zweiten Magneten (12b) positioniert ist und diese trennt, wodurch verhindert wird, dass die Innenkante (20a; 38a) des ersten Magneten (12a) die Innenkante (20b; 38b) des zweiten Magneten (12b) direkt berührt, wenn die magnetische Wundverschlussvorrichtung (10) entlang der Ränder einer Wunde (13) platziert wird;
wobei das längliche Trennelement (28) ein länglicher Streifen mit einer Breite von weniger als 2 cm, einer Tiefe von weniger als 2 cm ist und wobei der erste Magnet (12a) und der zweite Magnet (12b) eine Tiefe von weniger als 2 cm aufweisen;
wobei der Abstand zwischen der Innenkante (20a; 38a) des ersten Magneten (12a) und der Innenkante (20b; 38b) des zweiten Magneten (12b) weniger als 2 cm beträgt, wenn der längliche Streifen zwischen der Innenkante (20a; 38a) des ersten Magneten (12a) und der Innenkante (20b; 38b) des zweiten Magneten (12b) positioniert ist; und
wobei der erste und der zweite Magnet (12a, 12b) magnetisch zueinander gezogen werden, wenn die ablösbare Folie (26) und das längliche Trennelement (28) von dem ersten und dem zweiten Magneten (12a, 12b) entfernt werden, wodurch die Innenkante (20a; 38a) des ersten Magneten (12a) veranlasst wird, sich in Richtung der Innenkante (20b; 38b) des zweiten Magneten (12b) zu bewegen, wodurch die Ränder der Wunde (13) wieder angenähert werden.

2. Magnetische Wundverschlussvorrichtung (10) gemäß Anspruch 1, wobei die unteren Oberflächen (14a, 14b) des ersten Magneten (12a) und des zweiten Magneten (12b) jeweils eine Klebeschicht zum Befestigen des ersten Magneten (12a) und des zweiten Magneten (12b) auf die Haut eines Patienten aufweisen; und,
wobei die Klebstoffschicht durch eine ablösbare Klebeschichtabdeckung geschützt ist, die so konstruiert ist, dass sie abgelöst wird, bevor der erste Magnet (12a) und der zweite Magnet (12b) an der Haut eines Patienten angebracht werden.

3. Magnetische Wundverschlussvorrichtung (10) gemäß Anspruch 1 oder 2, wobei die ablösbare Folie (26) eine abziehbare Folie mit einer Klebeschicht auf der Unterseite (22) der ablösbaren Folie (26) zum Befestigen der ablösbaren Folie (26) am ersten Magneten (12a) und am zweiten Magneten (12b) ist.

4. Magnetische Wundverschlussvorrichtung (10) gemäß einem der vorangehenden Ansprüche, wobei der erste Magnet (12a) und der zweite Magnet (12b) flexible Magnete sind, die sich in Längsrichtung biegen können; und,
wobei die ablösbare Folie (26) sich in Längsrichtung biegen kann.

5. Magnetische Wundverschlussvorrichtung (10) gemäß einem der vorangehenden Ansprüche, wobei der erste Magnet (12a) und der zweite Magnet (12b) jeweils in einer Schutzhülle eingeschlossen sind.

6. Magnetische Wundverschlussvorrichtung (10) gemäß Anspruch 5, wobei die Schutzhülle eine Polymerhülle ist.

7. Magnetische Wundverschlussvorrichtung (10) gemäß einem der vorangehenden Ansprüche, wobei der längliche Streifen eine Breite von weniger als 1 cm, eine Tiefe von weniger als 1 cm aufweist und wobei der erste Magnet (12a) und der zweite Magnet (12b) jeweils eine Tiefe von weniger als 1 cm aufweisen.

8. Magnetische Wundverschlussvorrichtung (10) gemäß einem der vorangehenden Ansprüche, wobei die Unterseite (22) der ablösbaren Folie (26) im Wesentlichen die gleiche Größe und Form hat wie die oberen Oberflächen (24a, 24b) des ersten und zweiten Magneten (12a, 12b), wenn der erste und der zweite Magnet (12a, 12b) entlang ihrer jeweiligen Innenkanten (20a, 20b; 38a, 38b) aufeinander ausgerichtet sind; und
wobei das längliche Trennelement (28) im Wesentlichen entlang der Mitte der Längsachse der entfernbaren Folie (26) positioniert ist.

9. Magnetische Wundverschlussvorrichtung (10) gemäß einem der vorangehenden Ansprüche, wobei der erste und der zweite Magnet (12a, 12b) Neodym-Magnete sind.

10. Magnetische Wundverschlussvorrichtung (10) gemäß einem der vorangehenden Ansprüche, wobei sowohl der erste als auch der zweite Magnet (12a, 12b) Abmessungen im Bereich zwischen 5 cm und 20 cm Länge und 1 cm und 6 cm Breite aufweisen.

11. Magnetische Wundverschlussvorrichtung (10) gemäß einem der vorangehenden Ansprüche, wobei
die Innenkante (20a; 38a) des ersten Magneten (12a) eine erste Vielzahl von Kerben (30a) und eine erste Vielzahl von Vorsprüngen (32a) aufweist und die Innenkante (20b; 38b) des zweiten Magneten (12b) eine zweite Vielzahl von Kerben (30b) und eine zweite Vielzahl von Vorsprüngen (32b) aufweist und komplementär zur Innenkante (20a; 38a) des ersten Magneten (12a) geformt ist;
wobei der erste und der zweite Magnet (12a, 12b) erst ineinandergreifen, nachdem die ablösbare Folie (26) von den oberen Oberflächen (24a, 24b) des ersten und des zweiten Magneten (12a, 12b) entfernt wurde, wenn die erste Vielzahl von Kerben (30a) des ersten Magneten (12a) auf die zweite Vielzahl von Vorsprüngen (32b) auf dem zweiten Magneten (12b) ausgerichtet sind, wobei der erste und der zweite Magnet (12a, 12b) ein längsgerichtetes Gleiten entlang der Innenkanten (20a, 20b; 38a, 38b) des ersten Magneten (12a) und des zweiten Magneten (12b) verhindern und
wobei Wundränder wieder angenähert werden, wenn die magnetische Wundverschlussvorrichtung (10) entlang der Wundränder eines Patienten platziert wird, indem die Innenkante (20a; 38a) des ersten Magneten (12a) in Richtung der Innenkante (20b; 38b) des zweiten Magneten (12b) bewegt wird.

12. Magnetische Wundverschlussvorrichtung (10) gemäß Anspruch 11, wobei die erste Vielzahl von Kerben (30a) und die erste Vielzahl von Vorsprüngen (32a) Kerbverzahnungen sind.

13. Magnetische Wundverschlussvorrichtung (10) nach Anspruch 11 oder 12, wobei die erste und zweite Vielzahl von Kerben (30a, 30b) und die erste und zweite Vielzahl von Vorsprüngen (32a, 32b) V-förmig sind.

14. Magnetische Wundverschlussvorrichtung (10) nach einem der Ansprüche 11 bis 13, wobei die ablösbare Folie (26) so konstruiert ist, dass sie den ersten Magneten (12a) indirekt mit dem zweiten Magneten (12b) verbindet, indem sie an der oberen Oberfläche (24a) des ersten Magneten (12a) und der oberen Oberfläche (24b) des zweiten Magneten (12b) haftet.

## Revendications

1. Dispositif de fermeture de plaque magnétique (10) comprenant :
i) un premier aimant (12a) et un second aimant (12b), le premier aimant (12a) et le second aimant (12b) étant des aimants allongés ayant chacun une surface supérieure (24a, 24b), une surface inférieure (14a, 14b), un bord intérieur (20a, 20b ; 38a, 38b) et un bord extérieur (18a, 18b), dans lequel le bord intérieur (20a ; 38a) du premier aimant (12a) a une polarité opposée au bord intérieur (20b ; 38b) du second aimant (12b) ;
ii) une feuille amovible (26) ayant un élément de séparation allongé (28), la feuille amovible (26) étant conçue pour positionner le premier aimant (12a) par rapport au second aimant (12b) en adhérant à la surface supérieure (24a,) du premier aimant (12a) et à la surface supérieure (24b) du second aimant (12b) ;
dans lequel la feuille amovible (26) a une surface supérieure (27) et une surface inférieure (22), dans lequel la surface inférieure (22) de la feuille amovible (26) est fixée de façon amovible à la surface supérieure (24a) du premier aimant (12a) et à la surface supérieure (24b) du second aimant (12b) ;
dans lequel l'élément de séparation allongé (28) s'étend depuis la surface inférieure (22) de la feuille amovible (26) et est conçu pour être positionné entre, et pour séparer, le bord intérieur (20a ; 38a) du premier aimant (12a) et le bord intérieur (20b ; 38b) du second aimant (12b), empêchant entre le bord intérieur (20a ; 38a) du premier aimant (12a) d'entrer directement en contact avec le bord intérieur (20b ; 38b) du second aimant (12b) lorsque le dispositif de fermeture de plaque magnétique (10) est placé le long des bords d'une plaque (13) ;
dans lequel l'élément de séparation allongé (28) est une languette allongée ayant une largeur inférieure à 2 cm, une profondeur inférieure à 2 cm et dans lequel le premier aimant (12a) et le second aimant (12b) ont une profondeur inférieure à 2 cm ;
la distance entre le bord intérieur (20a ; 38a) du premier aimant (12a) et le bord intérieur (20b ; 38b) du second aimant (12b) étant inférieure à 2 cm lorsque la languette allongée est positionnée entre le bord intérieur (20a ; 38a) du premier aimant (12a) et le bord intérieur (20b ; 38b) du second aimant (12b) ; et lorsque la feuille amovible (26) et l'élément de séparation allongé (28) sont retirés des premier et second aimants (12a, 12b), les premier et second aimants (12a, 12b) étant attirés magnétiquement l'un vers l'autre, amenant le bord intérieur (20a ; 38a) du premier aimant (12a) à se déplacer en direction du bord intérieur (20b ; 38b) du second aimant (12b), rapprochant ainsi la plaque (13) des bords.

2. Dispositif de fermeture de plaque magnétique (10) selon la revendication 1, dans lequel les surfaces inférieures (14a, 14b) du premier aimant (12a) et du second aimant (12b) ont chacune une couche adhésive pour fixer le premier aimant (12a) et le second aimant (12b) à la peau d'un patient ; et
dans lequel la couche adhésive est protégée par un revêtement de couche adhésive amovible conçu pour être retiré avant fixation du premier aimant (12a) et du second aimant (12b) à la peau d'un patient.

3. Dispositif de fermeture de plaque magnétique (10) selon la revendication 1 ou 2, dans lequel la feuille amovible (26) est une feuille détachable ayant une couche adhésive sur la surface inférieure (22) de la feuille amovible (26) pour fixer la feuille amovible (26) au premier aimant (12a) et au second aimant (12b).

4. Dispositif de fermeture de plaque magnétique (10) selon l'une quelconque des revendications précédentes, dans lequel le premier aimant (12a) et le second aimant (12b) sont des aimants flexibles capables de fléchir dans une direction longitudinale ; et
dans lequel la feuille amovible (26) est capable de fléchir dans une direction longitudinale.

5. Dispositif de fermeture de plaque magnétique (10) selon l'une quelconque des revendications précédentes, dans lequel le premier aimant (12a) et le second aimant (12b) sont chacun insérés à l'intérieur d'un revêtement protecteur.

6. Dispositif de fermeture de plaque magnétique (10) selon la revendication 5, dans lequel le revêtement protecteur est une enceinte polymère.

7. Dispositif de fermeture de plaque magnétique (10) selon l'une quelconque des revendications précédentes, dans lequel la languette allongée a une largeur inférieure à 1 cm, une profondeur inférieure à 1 cm et dans lequel le premier aimant (12a) et le second aimant (12b) ont chacun une profondeur inférieure à 1 cm.

8. Dispositif de fermeture de plaque magnétique (10) selon l'une quelconque des revendications précédentes, dans lequel la surface inférieure (22) de la feuille amovible (26) est dimensionnée et formée sensiblement comme les surfaces supérieures (24a, 24b) des premier et second aimants (12a, 12b) lorsque les premier et second aimants (12a, 12b) sont orientés ensemble le long de leurs bords intérieurs (20a, 20b ; 38a, 38b) respectifs ; et
dans lequel l'élément de séparation allongé (28) est positionné sensiblement le long du centre de l'axe longitudinal de la feuille amovible (26).

9. Dispositif de fermeture de plaque magnétique (10) selon l'une quelconque des revendications précédentes, dans lequel les premier et second aimants (12a, 12b) sont des aimants en néodyme.

10. Dispositif de fermeture de plaque magnétique (10) selon l'une quelconque des revendications précédentes, dans lequel chacun des premier et second aimants (12a, 12b) a des dimensions dans la plage comprise entre 5 cm et 20 cm de long et 1 cm et 6 cm de large.

11. Dispositif de fermeture de plaque magnétique (10) selon l'une quelconque des revendications précédentes, dans lequel :
le bord intérieur (20a ; 38a) du premier aimant (12a) a une première pluralité d'entailles (30a) et une première pluralité de saillies (32a) et dans lequel le bord intérieur (20b ; 38b) du second aimant (12b) a une seconde pluralité d'entailles (30b) et une seconde pluralité de saillies (32b) et est formé de façon complémentaire au bord intérieur (20a ; 38a) du premier aimant (12a) ;
lorsque la première pluralité d'entailles (30a) du premier aimant (12a) sont alignées avec la seconde pluralité de saillies (32b) sur le second aimant (12b), les premier et second aimants (12a, 12b) ne s'interverrouillant entre eux qu'une fois la feuille amovible (26) retirée des surfaces supérieures (24a, 24b) des premier et second aimants (12a, 12b), dans lequel les premier et second aimants (12a, 12b) empêchent tout glissement longitudinal le long des bords intérieurs (20a, 20b ; 38a, 38b) du premier aimant (12a) et du second aimant (12b) ; et
les bords de plaque étant rapprochés lorsque le dispositif de fermeture de plaque magnétique (10) est placé le long des bords de plaque d'un patient en déplaçant le bord intérieur (20a ; 38a) du premier aimant (12a) en direction du bord intérieur (20b ; 38b) du second aimant (12b).

12. Dispositif de fermeture de plaque magnétique (10) selon la revendication 11, dans lequel la première pluralité d'entailles (30a) et la première pluralité de saillies (32a) sont des dentelures.

13. Dispositif de fermeture de plaque magnétique (10) selon la revendication 11 ou 12, dans lequel la première et la seconde pluralité d'entailles (30a, 30b) et la première et la seconde pluralité de saillies (32a, 32b) sont en forme de V.

14. Dispositif de fermeture de plaque magnétique (10) selon l'une quelconque des revendications 11 à 13, dans lequel la feuille amovible (26) est conçue pour relier indirectement le premier aimant (12a) au second aimant (12b) en adhérant à la surface supérieure (24a) du premier aimant (12a) et à la surface supérieure (24b) du second aimant (12b).
